# EUROPEAN PATENT APPLICATION

(11) **EP 2 468 175 A1**
(43) Date of publication of application: **27.06.2012**
(21) Application number: 10306516.5
(22) Date of filing: 24.12.2010
(51) Int. Cl.: A61B 5/00, A61K 49/00

(54) **Method for managing the risk of liver injury in patients receiving treatment with dronedarone**

(71) Applicant: SANOFI, 75008 Paris (FR)
(72) Inventor: The designation of the inventor has not yet been filed
(74) Representative: Bouron, Estelle

(57) **Abstract**

Method to manage the risk of liver injury in patients receiving treatment with dronedarone or pharmaceutically acceptable salts thereof by performing the following steps: performing liver function tests prior to treatment with dronedarone, monitoring liver function monthly for six months, at months 9 and 12, and periodically thereafter, if alkaline phosphatase (ALT) levels are elevated higher than three times the upper limit of normal, re-measuring levels; if confirmed to be greater than three times the upper limit of normal, withdrawing administration of dronedarone, continuing close observation until normalization of ALT and, investigating the probable cause, including those related to underlying cardiac conditions.

## Description

The present invention relates to the method of managing the risk of liver injury in patients receiving treatment with dronedarone or pharmaceutically acceptable salts thereof.

The instant invention also relates to a method of reducing the risk of liver injury in patients receiving treatment with dronedarone or pharmaceutically acceptable salts thereof.

2-n-Butyl-3-[4-(3-di-n-butylaminopropoxy)benzoyl]-5-methylsulphonamidobenzofuran, or dronedarone, and pharmaceutically acceptable salts thereof, in particular its hydrochloride salts, are described in European Patent EP 0 471 609 B1.

Moreover, dronedarone is effective in maintaining sinus rhythm in patients presenting atrial fibrillation or atrial flutter.

The applicant has clinically proven that dronedarone significantly reduces cardiovascular hospitalizations and/or mortality in patients having a history of atrial fibrillation (AF) or of atrial flutter (AFL) in a safe and effective way. Dronedarone is indicated to reduce the risk of cardiovascular hospitalization in patients with paroxysmal or persistent AF or AFL, with a recent episode of AF/AFL and associated cardiovascular risk factors (i.e., age >70, hypertension, diabetes, prior cerebrovascular accident, left atrial diameter ≥50 mm or left ventricular ejection fraction [LVEF] <40%), who are in sinus rhythm or who will be cardioverted.

The Applicant has now found the regimen to administrate dronedarone to patients in a safe and effective way, with a method to manage the risk of liver injury in patients receiving treatment with dronedarone or pharmaceutically acceptable salts thereof by performing the following steps:
a) performing liver function tests prior to treatment with dronedarone,
b) monitoring liver function monthly for six months, at months 9 and 12, and periodically thereafter,
c) if alkaline phosphatase (ALT) levels are elevated higher than three times the upper limit of normal (ULN), re-measuring levels;
d) if confirmed to be greater than three times the upper limit of normal, withdrawing administration of dronedarone.
e) continuing close observation until normalization of ALT and,
f) investigating the probable cause, including those related to underlying cardiac conditions.

Liver injury may comprise hepatic events such as liver function test abnormalities and hepatocellular liver injury, including acute hepatic failure or life-threatening acute liver failure.

Liver functions test may comprise determination of liver enzymes levels. These enzymes may be alkaline phosphatase (ALP, AP), alanine aminotransferase (ALT), aspartate aminotransferase (AST), total bilirubine.

"Managing the risk" may be defined as "reducing the risk".

The methods according to the invention enable to decrease the risk of liver injury, when dronedarone or pharmaceutically acceptable salts or esters thereof is administered for treating patients with paroxysmal or persistent atrial fibrillation (AF) or atrial flutter (AFL), with a recent episode of AF/AFL and associated cardiovascular risk factors (i.e., age >70, hypertension, diabetes, prior cerebrovascular accident, left atrial diameter ≥50 mm or left ventricular ejection fraction [LVEF] <40%), who are in sinus rhythm or who will be cardioverted.

The patients concerned by the present invention have a cardiovascular history. They may be affected, for example, by a Atrial Fibrillation such as a persistent Atrial Fibrillation, a paroxysmal Atrial Fibrillation or a permanent Atrial Fibrillation or by a Atrial Flutter.

According to another embodiment, patients are chosen from patients with paroxysmal or persistent atrial fibrillation.

The American College of Cardiology, American Heart Association, and the European Society of Cardiology recommend in their guidelines the following classification system based on simplicity and clinical relevance:
➢ Patients with Paroxysmal Atrial Fibrillation means patients with recurrent episodes that self-terminate in less than 7 days.
➢ Patients with persistent Atrial Fibrillation means patients with recurrent episodes that last more than 7 days.

If a first detected episode self-terminates in less than 7 days and then another episode begins later on, the case has moved into the category of paroxysmal AF. Although patients in this category have episodes lasting up to 7 days, in most cases of paroxysmal AF the episodes will self-terminate in less than 24 hours. If instead the episode lasts for more than 7 days, it is unlikely to self-terminate and it is called persistent AF. In this case, the episode may be terminated by cardioversion.
➢ If cardioversion is unsuccessful or it is not attempted, and the episode is ongoing for a long time (e.g. a year or more), the patient's AF is called permanent.

Among the patients having a cardiovascular history according to the invention, in particular having a history of atrial fibrillation or atrial flutter, mention may also be made of patients also exhibiting at least one additional risk factors, such as:
- age equal to or above 70, or even above 75
- hypertension,
- diabetes,
- prior cerebrovascular disease,
- left atrial diameter greater than or equal to 50 mm measured by echocardiography,
- left ventricular ejection fraction less than 40%, measured by two-dimensional echography.

Among the patients having a cardiovascular history according to the invention, in particular having a history of atrial fibrillation or atrial flutter, mention may also be made of patients also exhibiting additional risk factors, i.e. at least one pathologies chosen from :
- Structural heart disease,
- Lone Atrial Fibrillation,
- Coronary heart disease, and
- Dilated cardiomyopathy,
   and/or at least one cardiac device chosen from:
- Pacemaker, and
- Valvular heart.

For their therapeutic use, dronedarone and pharmaceutically acceptable salts thereof are generally introduced into pharmaceutical compositions.

These pharmaceutical compositions contain an effective dose of dronedarone or of a pharmaceutically acceptable salt thereof, and also at least one pharmaceutically acceptable excipient.

Said excipients are chosen according to the pharmaceutical form and the method of administration desired, from the usual excipients which are known to those skilled in the art.

In said pharmaceutical compositions for oral, sublingual, subcutaneous, intramuscular, intravenous, topical, local, intratracheal, intranasal, transdermal or rectal administration, dronedarone, or the salt thereof, can be administered in unit administration form, as a mixture with conventional pharmaceutical excipients, to animals and to humans in the cases mentioned above.

The suitable unit administration forms comprise forms for oral administration, such as tablets, soft or hard gel capsules, powders, granules and oral solutions or suspensions, sublingual, buccal, intratracheal, intraocular or intranasal administration forms, forms for administration by inhalation, topical, transdermal, subcutaneous, intramuscular or intravenous administration forms, rectal administration forms, and implants. For topical application, dronedarone and pharmaceutically acceptable salts thereof can be used in creams, gels, ointments or lotions.

By way of example, a unit administration form of dronedarone or a pharmaceutically acceptable salt thereof, in tablet form, may correspond to one of the following compositions (Examples 1 - 4) according to the invention:

| **EXAMPLE 1** | |
|---|---|
| Ingredients | mg |
| Dronedarone hydrochloride (corresponding to 400 mg of base) | 426 |
| Methylhydroxypropylcellulose | 21.1 |
| Lactose monohydrate | 46.55 |
| Maize starch | 45.5 |
| Polyvinylpyrrolidone | 65 |
| Poloxamer 407 | 40 |
| Anhydrous colloidal silica | 2.6 |
| Magnesium stearate | 3.25 |
| | 650 |
| | |

| **EXAMPLE 2** | |
|---|---|
| Ingredients | mg |
| Dronedarone hydrochloride (corresponding to 400 mg of base) | 426 |
| Microcrystalline cellulose | 65 |
| Anhydrous colloidal silica | 2.6 |
| Anhydrous lactose | 42.65 |
| Polyvinylpyrrolidone | 13 |
| Poloxamer 407 | 40 |
| Macrogol 6000 | 57.5 |
| Magnesium stearate | 3.25 |
| | 650 |

| **EXAMPLE 3** | |
|---|---|
| Ingredients | mg |
| Dronedarone hydrochloride (corresponding to 400 mg of base) | 426 |
| Microcrystalline cellulose | 26 |
| Maize starch | 45.5 |
| Polyvinylpyrrolidone | 65 |
| Poloxamer 407 | 40 |
| Anhydrous colloidal silica | 3.25 |
| Magnesium stearate | 3.25 |
| Lactose monohydrate | 41.65 |
| | 650 |
| | |

| **EXAMPLE 4** | |
|---|---|
| Ingredients | mg |
| Dronedarone hydrochloride (corresponding to 400 mg of base) | 213 |
| Microcrystalline cellulose | 13 |
| Maize starch | 22.75 |
| Polyvinylpyrrolidone | 32.5 |
| Poloxamer 407 | 20 |
| Anhydrous colloidal silica | 1.3 |
| Magnesium stearate | 1.625 |
| Lactose monohydrate | 20.825 |
| | 650 |

The dose of dronedarone administered per day, orally, may reach 800 mg, taken in one or more intakes. More specifically, the dose of dronedarone administered may be taken with food. For example, the dose of dronedarone administered per day, orally, may reach 800 mg, taken in two intakes with a meal.

The dose of dronedarone administered per day, orally may be taken at a rate of twice a day (usually abbreviated BID) with a meal for example with the morning and the evening meal. More specifically, the two intakes may comprise same quantity of dronedarone.

Advantageously, the dose of dronedarone administered, orally, may reach 400 mg BID, taken together with a meal, for example with the morning and the evening meal.

There may be specific cases where higher or lower dosages are appropriate; such dosages do not depart from the context of the invention. According to the usual practice, the dosage appropriate for each patient is determined by the physician according to the method of administration, the weight, the pathology, the body surface, the cardiac output and the response of said patient.

According to another of its aspects, the present invention also relates to a method for treating the pathologies indicated above, which comprises the administration, to a patient, of an effective dose of dronedarone or a pharmaceutically acceptable salt thereof.

The present invention is illustrated by the data hereinafter with reference to the attached drawings in which:

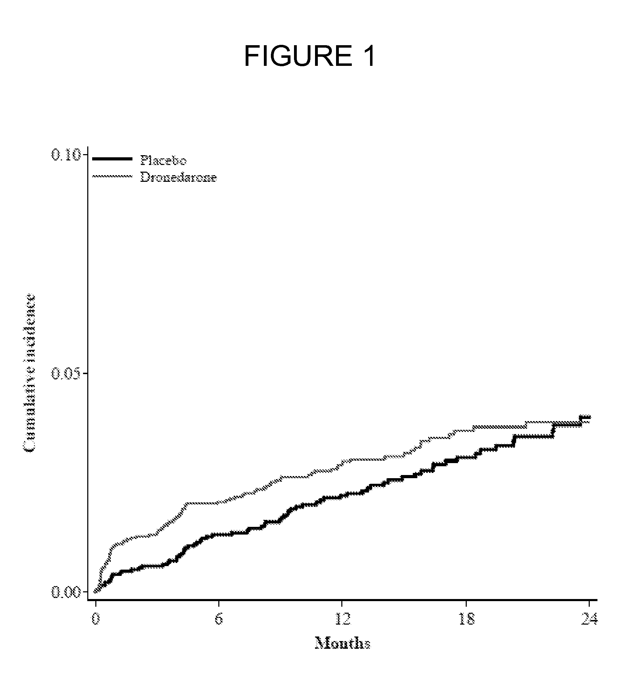

Figure 1 represents a Kaplan Meier curve with the cumulative rate of first hepatic event or ALT greater than or equal to 5 times ULN during the on-treatment period.

In the pool of 5 placebo controlled studies in patients with AF/AFL (DRI3550/DAFNE, EFC4508/ERATO, EFC3153/EURIDIS, EFC4788/ADONIS, EFC5555/ATHENA), the hepatic events were analyzed with the following selection:
- ALT ≥ 5 x ULN, or
- SOC 'Hepatobiliary disorders', or
- SMQ "Liver related investigations signs and symptoms" using broad and narrow selection

The time to onset analysis of these hepatic events showed a trend in which the dronedarone group had an earlier onset of hepatic events during the first 6 months compared to placebo (Figure 1).

After 1 year, the incidences appeared to be similar in the 2 groups, the data being entirely driven by adverse events reported in the ATHENA study (enzymes not routinely collected).

These events occurred within 6 months after dronedarone treatment initiation and consequently support the claimed method to monitor liver function and risk of liver injury.

## Claims

1. Method to manage the risk of liver injury in patients receiving treatment with dronedarone or pharmaceutically acceptable salts thereof by performing the following steps:
a) performing liver function tests prior to treatment with dronedarone,
b) monitoring liver function monthly for six months, at months 9 and 12, and periodically thereafter,
c) if alkaline phosphatase (ALT) levels are elevated higher than three times the upper limit of normal (ULN), re-measuring levels;
d) if confirmed to be greater than three times the upper limit of normal, withdrawing administration of dronedarone.
e) continuing close observation until normalization of ALT and,
f) investigating the probable cause, including those related to underlying cardiac conditions.

2. Method according to claim 1 wherein liver injury comprise hepatocellular liver injury.

3. Method according to claim 1 wherein liver injury comprise life-threatening acute liver failure.

4. Method according to anyone of claims 1 to 3 wherein liver functions test comprise determination of liver enzymes levels.

5. Method according to anyone of the preceding claims, **characterized in that** patients have persistent Atrial Fibrillation.

6. Method according to anyone of the preceding claims, **characterized in that** patients have paroxysmal Atrial Fibrillation.

7. Method according to anyone of the preceding claims, **characterized in that** patients have permanent Atrial Fibrillation.

8. Method according to anyone of the preceding claims, **characterized in that** the dose of dronedarone administered orally, is 400 mg BID.

9. Method according to anyone of the preceding claims, **characterized in that** patients also exhibit at least one additional cardiovascular risk factor chosen from:
- age equal to or above 70, or even above 75,
- hypertension,
- diabetes,
- prior cerebrovascular disease,
- left atrial diameter greater than or equal to 50 mm measured by echocardiography, and
- left ventricular ejection fraction less than 40%, measured by two-dimensional echography.

10. Method according to anyone of the preceding claims, **characterized in that** patients also exhibit at least one pathologies chosen from :
- Structural heart disease,
- Lone Atrial Fibrillation,
- Coronary heart disease, and
- Dilated cardiomyopathy,
and/or at least one cardiac device chosen from:
- Pacemaker, and
- Valvular heart.
